# EUROPÄISCHE PATENTSCHRIFT

(11) **EP 1 624 903 B1**
(45) Veröffentlichungstag und Bekanntmachung des Hinweises auf die Patenterteilung: **14.03.2007**
(21) Anmeldenummer: 03735395.0
(22) Anmeldetag: 15.05.2003
(51) Int. Cl.: A61L 2/28, B65D 79/02, B65D 81/00

(54) **TESTEINHEIT UND VERFAHREN ZUR PRÜFUNG EINER STERILISIERVERPACKUNGSEINHEIT AUF WIRKSAMKEIT GEGEN REKONTAMINATION UND EINE ZU DEM VERFAHREN GEEIGNETE CONTAINERVERPACKUNG**
TEST UNIT AND METHOD FOR TESTING WHETHER A STERILIZING PACKAGING UNIT IS EFFECTIVE AGAINST RECONTAMINATION, AND CONTAINER PACKAGING SUITABLE FOR APPLYING SAID METHOD
UNITE DE VERIFICATION ET PROCEDE PERMETTANT DE VERIFIER L'EFFICACITE D'UNE UNITE D'EMBALLAGE DE STERILISATION CONTRE LA RECONTAMINATION, ET EMBALLAGE DE TYPE CONTENEUR SERVANT A LA MISE EN OEUVRE DUDIT PROCEDE

(43) Veröffentlichungstag der Anmeldung: 15.02.2006
(73) Patentinhaber: DUNKELBERG, Hartmut, 37242 Bad Sooden-Allendorf (DE)
(72) Erfinder: DUNKELBERG, Hartmut, 37242 Bad Sooden-Allendorf (DE)
(74) Vertreter: Callies, Rainer Michael
(86) Internationale Anmeldenummer: PCT/EP2003/005111
(87) Internationale Veröffentlichungsnummer: WO 2004/101006

(56) Entgegenhaltungen:
- GB-A- 2 186 974
- US-A- 4 596 773
- US-A- 5 167 923
- US-A- 5 344 017
- US-A1- 2002 022 246
- JUNGHANNSS U ET AL: "HYGIENISCH-MIRKOBIOLOGISCHE UND TECHNISCHE UEBERPRUEFUNGEN VON STERILISIER-CONTAINERSYSTEMEN HYGIENIC-MICROBIOLOGICAL AND TECHNICAL TESTING OF STERILISER CONTAINER SYSTEMS" ZENTRAL STERILISATION, XX, XX, Bd. 7, Nr. 3, 1999, Seiten 154-162, XP001153080

## Beschreibung

Die Erfindung bezieht sich auf eine Testeinheit und ein Verfahren zur Prüfung einer zur Sterilisation von Sterilisiergut, insbesondere medizinischem Sterilisiergut, vorgesehenen Sterilisierverpackungseinheit hinsichtlich der Wirksamkeit der Sterilisierverpackungseinheit, eine mikrobielle Rekontamination des Sterilisiergutes nach dessen erfolgter Sterilisation zu verhindern. Ferner bezieht sich die Erfindung auf eine besondere Sterilisierverpackungseinheit, nämlich eine, die zumindest teilweise eine starre Außenhülle aufweist. Die letztgenannte Sterilisierverpackungseinheit ermöglicht es, das Verfahren zur Prüfung hinsichtlich der Wirksamkeit gegen Rekontamination auch auf solch eine Sterilisierverpackungseinheit anzuwenden.

Unter Sterilisierverpackungseinheit wird die Verpackung an sich, also ohne einen Inhalt verstanden. Zum Beispiel kann eine Sterilisierverpackungseinheit eine so genannte Primärpackung sein, welche ein versiegeltes oder verschlossenes Verpackungssystem zur Umschließung des Sterilisiergutes ist. Unter Verpackungssystem werden ein oder mehrere Verpackungsmaterialien verstanden, die als Teil oder Ganzes einer Primärpackung bestimmt sind. Die Sterilisierverpackungseinheit kann auch eine so genannte Sekundärpackung sein, die zur Aufnahme eines oder mehrerer Sterilisiergutteile, jedes in seiner Primärpackung, vorgesehen ist. Unter Sterilisierverpackungseinheit soll insbesondere eine Endpackung verstanden werden. Hierbei handelt es sich um die Packung, in der insbesondere ein Medizinprodukt sterilisiert wird. Die Endpackung kann eine Primärpackung sein, zu der zusätzlich eine Sekundär- und/oder Transportpackung vorgesehen ist.

Insbesondere im medizinischen Bereich müssen viele Gegenstände vor ihrer Benutzung sterilisiert werden, um auf den Gegenständen vorhandene Keime, d.h. lebensfähige Mikroorganismen, abzutöten. Bei solchem medizinischem Sterilisiergut handelt es sich beispielsweise um Instrumente, Wäsche oder Flüssigkeiten. Je nach Art des Sterilisiergutes wird ein entsprechendes Sterilisationsverfahren physikalischer oder chemischer Art angewandt. Im medizinischen Bereich werden vor allem Dampfsterilisationsverfahren angewandt. Diese zeichnen sich durch eine hohe Wirksamkeit und Umweltverträglichkeit aus. Bei Dampfsterilisationsverfahren wird mit gespanntem und gesättigtem Wasserdampf sterilisiert. Die Temperatur des Wasserdampfes beträgt dabei beispielsweise 121 °C oder 134 °C. Die Sterilisation findet mittels Autoklaven statt.

Weiterhin können Keime auch durch energiereiche ionisierende Strahlen oder durch ein Plasma auf H₂O₂-Basis abgetötet werden. Zu den chemischen Sterilisationsverfahren zählen die Sterilisation mittels Ethylenoxid oder Förmaldehyd.

Zur Sterilisation wird medizinisches Sterilisiergut verpackt. Dazu können Containerverpackungen, beispielsweise in Form eines Koffers, Papierverpackungen, bei denen das Sterilisiergut mehrfach in Papier eingeschlagen wird, und andere Verpackungen verwendet werden. Papierverpackungen mit Klarsichthülle werden industriell zur Verpackung von sterilem Einwegmaterial und im Krankenhaus zur Einzelverpackung von Einwegmaterial oder wiederverwendbaren Gütern eingesetzt. Bei diesen Verpackungen kann es sich insbesondere um Verpackungen auf der Grundlage von Klarsichtbeuteln und Klarsichtschläuchen aus Papier und Kunststoff-Verbundfolie handeln. Die Verpackung dient dazu, die durch den Sterilisationsvorgang erreichte Keimfreiheit bis zum Gebrauch des Sterilisiergutes zu gewährleisten. Die Lagerzeit im Krankenhaus beträgt Wochen bis Monate.

Die Qualitätsanforderungen an den Sterilisationsvorgang sind hoch. Entsprechend WHO-Richtlinien und verschiedenen europäischen Arzneibüchern müssen von 1 000 000 "sterilen" Produkten 999 999 keimfrei sein. Diese Anforderung ist der wesentliche Inhalt der Europäischen Norm EN 556-1:2001.

Der Sterilisationsvorgang selbst wird üblicherweise mit physikalisch-chemischen und mikrobiologischen Parametern evaluiert und standardisiert. Ein bekanntes und verlässliches Verfahren zur Prüfung der Wirksamkeit eines Sterilisationsvorgangs besteht darin, gleichzeitig mit dem Sterilisiergut auch eine Testkeimeinheit dem zu prüfenden Sterilisationsvorgang zu unterziehen. Bei dieser Testkeimeinheit handelt es sich um eine Anzahl von gegenüber dem Sterilisationsprozess besonders resistenten Keimen, die als Einheit durch eine Hülle verschlossen sind. Nach dem Sterilisationsvorgang wird diese Testkeimeinheit unter sterilen Bedingungen geöffnet, und es wird geprüft, ob die darin enthaltenen Keime vermehrungsfähig sind. Wenn der Sterilisationsvorgang ordnungsgemäß erfolgt ist, wird keine Vermehrung der Testkeime stattfinden, da diese Testkeime durch die Sterilisation abgetötet worden sind. Die Testkeime stellen somit einen Indikator für die Effektivität des Sterilisationsvorgangs dar.

Eine solche Testkeimeinheit gibt es insbesondere in Form von selbstentwickelnden Bio-Indikatoren (erhältlich von der Firma 3M Medica, Zweigniederlassung der 3M Deutschland GmbH, Neuss, Deutschland). Sie weisen eine Nährmedium enthaltende Ampulle sowie eine Plastikhülle auf, in der sich die Ampulle sowie Sporen definierter Keimart befinden. Die Plastikhülle besitzt einen Verschluß mit einem dampfdurchlässigen, aber keimdichten Filter. Die Bio-Indikatoren werden dem zu prüfenden Sterilisationsprozess unterzogen. Anschließend wird die Ampulle zerdrückt. Dadurch vermischen sich die Keime und das Nährmedium. Danach wird der Indikator im Inkubator bebrütet und hinsichtlich eines erfolgten Farbumschlags des Nährmediums ausgewertet. Wenn kein Farbumschlag vorliegt, fand kein Keimwachstum statt. In diesem Fall ist der Sterilisationsvorgang effizient gewesen und hat die Keime des Bio-Indikators abgetötet. Ein ähnlicher Indikator ist auch von der Firma gke-mbH, Waldems-Esch, Neuss, Deutschland, erhältlich. Mit solchen Indikatoren kann jedoch konstruktionsbedingt nicht geprüft werden, ob nach einer erfolgreichen Sterilisation während einer bestimmten Lagerungszeitdauer eine Rekontamination des Sterilisiergutes eingetreten ist.

Auch für das Verpackungsmaterial gibt es Qualitätsnormen. Diese beziehen sich in der Regel auf Teilaspekte der Sterilisierverpackungseinheit wie Materialeigenschaften, Dichtigkeit von Verschlüssen, usw.

Bei einer Rekontamination des Sterilisiergutes spielt zum einen die so genannte Keimdichtigkeit der verwendeten Sterilisierverpackungseinheit eine Rolle. Diese Keimdichtigkeit ist die Fähigkeit der Sterilisierverpackungseinheit, den Eintritt von Mikroorganismen zu verhindern. Zum anderen spielen für eine Rekontamination verschiedene äußere Einflüsse wie die Art der Lagerung oder des Transports und mechanische Beanspruchung des verpackten Sterilisiergutes sowie Umgebungseinflüsse wie Luftbewegung, Luftkeimgehalt und Luftdruckschwankungen eine Rolle.

Eine bekannte Vorrichtung zur Prüfung von Teilkomponenten einer Containerverpackung auf Keimdichtigkeit ist so ausgebildet, dass sie über in einem Deckel des Containers vorhandene Durchlässe einen Unterdruck in dem Container erzeugt (EN 868-1, Anhang G). Mittels dieses Unterdrucks kann die Dichtheit einer Dichtung zwischen dem Deckel und der Containerwanne getestet werden. Hierbei handelt es sich jedoch lediglich um eine Vorrichtung für ein spezielles physikalisches Prüfverfahren für Verpackungen in Gestalt von Containern. Dieses Verfahren bezieht sich nur auf die Dichtung zwischen Wanne und Deckel, nicht jedoch auf die Keimdichtigkeit der Dampfdurchlässe wie Filter und Ventile. Darüber hinaus sind bei diesem Prüfverfahren Umgebungseinflüsse, wie z.B. der Luftkeimgehalt, nicht einbezogen. Somit kann mittels dieser Vorrichtung keine sichere Aussage darüber getroffen werden, inwieweit mit einer Rekontamination von in der Sterilisierverpackungseinheit enthaltenem Sterilisiergut gerechnet werden muss.

Ferner gibt es eine mikrobiologische DIN-Prüfvorrichtung (DIN 58953, Teil 6), die zur Prüfung von Sterilisationspapier vorgesehen ist. Bei dieser Vorrichtung wird ein Keimdurchlässigkeitsprüfgerät in Form einer Laborglasflasche mit dem zu prüfenden Sterilisationspapier verschlossen und geprüft, ob durch Abkühlung der Luft im Keimdurchlässigkeitsgerät ein Luftstrom in die Laborglasflasche gelangt, der keimhaltige Partikel durch das Sterilisationspapier transportiert, wenn dieses vor dem Abkühlen mit einem eine definierte Keimzahl von Bacillus subtilis-Sporen enthaltenden Pulver beschichtet wurde. Dabei ist vorgesehen, die Laborglasflasche mehrfach hintereinander auf 50 °C zu erwärmen und auf 10 °C abzukühlen. Eventuell durch das Sterilisationspapier in die Laborglasflasche eintretende Sporen werden mikrobiologisch mittels Bebrütung der Nährmedium enthaltenden Laborglasflasche erfasst und ausgewertet. Diese Prüfung bezieht sich jedoch nicht auf eine Sterilisierverpackungseinheit als Ganzes, sondern nur auf Papier, das als Material für Verpackungen verwendet wird. Es handelt sich um eine reine Materialprüfung, bei der abgesehen von dem Aufbringen des Pulvers keine weiteren Umgebungseinflüsse einbezogen werden.

Aus der Publikation von de Bruijn, A.C.P. und Kastelein, J., Einfach- oder Mehrfach-Verpackung von Medizinprodukten: Verfahrensbewertung durch Forschung, Zentralsterilisation 1999; 7 (5): 292-303 ist eine Vorrichtung bekannt, mit der die Filterwirkung einer Verpackung gegenüber einem Aerosol aus 1,0 µm Latexpartikeln mittels eines Partikelzählgerätes (vgl. a.a.O., 3., S. 297, 1. Abs.) bestimmt wird. Die bekannte Vorrichtung ist nicht dazu bestimmt und nicht geeignet, Verpackungen in ihrer Gebrauchsform auf ihr Vermögen zu untersuchen, Sterilität unter Bedingungen der Krankenhauspraxis zu erhalten. Insbesondere beruht das der Vorrichtung zugrunde liegende Verfahren nicht auf der Bestimmung des relevanten Endpunktes, nämlich der Erfassung der mikrobiologischen Kontamination.

Von der American Society for Testing and Materials (ASTM) wird unter Designation F 1608-00 ein Verfahren beschrieben, mit dem die Keimdurchlässigkeit bzw. Barrierewirksamkeit von porösen Materialien wie Papier bestimmt wird: In eine Kammer wird durch einen Vernebler ein keimhaltiges Aerosol mit Bacillus subtilis-Sporen eingebracht. Unter Zwischenschaltung des Prüfmaterials auf Filterträger und einer weiteren keimdichten Filtermembran kann die Keimdurchlässigkeit des Prüfkörpers bei definierter Passage eines keimhaltigen Aerosols bestimmt werden. Auch in diesem Fall handelt es sich um eine Vorrichtung zur Prüfung eines zur Verpackung eingesetzten Materials, nicht aber um eine Vorrichtung zur Untersuchung einer praxisüblichen Sterilisierverpackungseinheit.

Weiter ist bekannt, stichprobenhaft Teile des Sterilisiergutes nach einer bestimmten Zeitspanne auf Rekontamination zu testen. Nachteilig hierbei ist jedoch die Tatsache, dass dazu die Sterilisierverpackungseinheit geöffnet werden muss und bei der Untersuchung selbst Keime auf den Untersuchungsgegenstand gelangen können, so dass das Untersuchungsergebnis dadurch fehlerbehaftet sein kann. Für den Krankenhausbetrieb ist dieses Verfahren jedoch nicht praktikabel, so dass es routinemäßig in Krankenhäusern keine Anwendung findet.

Aus der Publikation von Junghannß U., Winterfeld S., Gabele L., Kulow U., Hygienisch-mikrobiologische und technische Überprüfungen von Sterilisier-Containersystemen, Zentralsterilisation 1999; 7 (3): 154-162 ist eine Vorrichtung zur Prüfung von Sterilisierbehältern in Form von Verpackungscontainern bekannt. Diese Vorrichtung besteht aus einer Testkammer, in welche die zu prüfenden Container eingebracht werden können. Vor Einbringung in die Testkammer wurden die Container mit den dazugehörigen Filtern beschickt und sterilisiert. Anschließend wurden die Container mit Nährboden in Petrischalen versehen und wieder verschlossen. Mittels einer Sprühflasche wurde in das Innere der Testkammer ein keimhaltiges Aerosol gegeben. Über einen Anschlussstutzen, mit dem die zu prüfenden Container vor Einbringung in die Testkammer versehen worden waren, erfolgte eine Absaugung mittels einer Schlauchpumpe durch die zu überprüfenden Container hindurch. Nach dem Absaugen wurden die Container wiederum geöffnet, und die Nährbodenschalen wurden entnommen. Anschließend wurden diese zum Nachweis von Keimen auf dem Nährboden bebrütet. Daran anschließend erfolgte das Auszählen von koloniebildenden Einheiten (KBE).

Die bekannte Vorrichtung eignet sich nicht dazu, eine Endpackung in der Form, wie sie in der Praxis eingesetzt wird, auf ihre Barrierewirksamkeit bezüglich einer Rekontamination zu prüfen. Vielmehr müssen die Container vor Einbringung in die bekannte Testkammer mit einem Ansaugstutzen versehen werden. Ferner müssen die Container, nachdem sie in der Testkammer in Kontakt mit dem Aerosol waren, aus der Testkammer herausgenommen und geöffnet werden, um die Nährbodenschalen zu entnehmen und in einer separaten Vorrichtung zu bebrüten. Dadurch ist verhindert, dass der Container im ungestörten Zustand bis zur Auswertung der Untersuchungsergebnisse bleibt. Somit sind äußere Fehlerquellen bei der Untersuchung nicht auszuschließen. Die vorgesehene Entnahme der Nährbodenschalen für die nachfolgende Bebrütung ist vielmehr mit einem Kontaminationsrisiko und somit mit dem Risiko einer Messungenauigkeit verbunden. Nach Junghannß et al. wird lediglich ein Differenzwert für die Keimundurchlässigkeit bestimmt, jedoch kein Absolutwert ermittelt. Bei der bekannten Versuchsanordnung konnte keine Aussage zur Keimfreiheit (Sterilität) aufgrund des unsterilen Umgangs mit dem Nährboden gemacht werden.

In der Europäischen Norm EN 868-1 von 1997 ist unter Punkt 4.6 ausgeführt, dass es an einem geeigneten Endpackungs-Prüfverfahren, welches genormt ist, mangelt. Ein solches Verfahren gibt es auch heute noch nicht. So wird auch in dem "ASTM-Standard Guide for Design and Evaluation of Primary Packaging for Medical Products" von 2001 (Designation: F 2097 - 01) unter Punkt 4.6.2 darauf hingewiesen, dass kein einzelnes Testverfahren vollständig die Leistungsfähigkeit der Endpackung vorhersagen könne.

Es ist bisher nur ein Verfahren vorgeschlagen worden, welches geeignet ist zur Prüfung der Verpackung als Ganzes bzw. einer in Gebrauch befindlichen Sterilisierverpackungseinheit hinsichtlich ihrer Wirksamkeit, in der Zeitspanne nach der Sterilisation bis zu dem Zeitpunkt des Gebrauchs des Sterilisiergutes dessen Rekontamination zu verhindern (Dunkelberg, H. und Wedekind, S., Hygiene + Medizin, 27. Jahrgang, 2002 - Suppl. 1, 29, Rekontamination von papierverpackten Sterilgutpaketen unter mechanischer Belastung; Dunkelberg, H. und Wedekind, S., Biomedizinische Technik, 47 (2002), 290-293, Eine neue Methode zur Wirksamkeitsprüfung von Sterilisiergutverpackungen in der Praxis). Mit dem Verfahren, das Gegenstand der Patentschrift EP-B-1 485 135 ist, kann auch eine möglicherweise erfolgte Rekontamination des Sterilisiergutes erfasst werden. Bei diesem bekannten Verfahren zur Prüfung einer zur Sterilisation von Sterilisiergut, insbesondere medizinischem Sterilisiergut, vorgesehenen Sterilisierverpackungseinheit hinsichtlich ihrer Wirksamkeit, eine mikrobielle Rekontamination des Sterilisiergutes nach dessen erfolgter Sterilisation zu verhindern, sind folgende Schritte vorgesehen: Die Sterilisierverpackungseinheit wird mit Nährmedium, z.B. mit Nähragar-befüllten Schalen, beladen. Anschließend wird die Sterilisierverpackungseinheit einem Sterilisationsvorgang unterzogen und danach unter Umgebungsbedingungen über einen Zeitraum gelagert, wobei grundsätzlich sofort nach dem Sterilisationsvorgang die Möglichkeit gegeben ist, dass sich Keime im Nährmedium niederlassen. Daran anschließend wird die Sterilisierverpackungseinheit Vermehrungsbedingungen von Keimen ausgesetzt zu einer Kultivierung von in oder auf dem Nährmedium vorhandenen Keimen. Schließlich wird die Sterilisierverpackungseinheit geöffnet und Keime, die sich in oder auf dem Nährmedium vermehrt haben, werden ermittelt.

Bei diesem bekannten Verfahren ist das Nährmedium sofort nach dem Sterilisationsvorgang gebrauchsfertig. Es können sich bereits zu diesem Zeitpunkt Keime in oder auf dem Nährmedium niederlassen und vemehren. Die Zeitdauer, während der ein Nährmedium als Grundlage für eine Vermehrung von Keimen eingesetzt werden kann, ist als Folge des unvermeidbaren Wasserverlustes begrenzt und beträgt beispielsweise für Nährboden höchstens zwei bis drei Wochen. Somit ist bei dem bekannten Verfahren der Zeitpunkt, zu dem noch eine erfolgte Rekontamination ermittelt werden kann, durch die begrenzte Haltbarkeitsdauer des Nährmediums limitierend vorgegeben.

Ferner ist eine Vorrichtung bekannt, mit der insbesondere das Verfahren gemäß Patentschrift EP-B-1 485 135, das zur Prüfung der Verpackung als Ganzes geeignet ist, durchgeführt werden kann (Dunkelberg, H., Zietz, B., Wedekind, S., The Society for Healthcare Epidemiology of America, 13^{th} Annual Scientific Meeting, Preliminary Results for a New Final Package Test to Assess the Quality of Sterile Package Systems). In diesem Fall können die Umgebungsbedingungen so festgelegt werden, dass die zu prüfende Sterilisierverpackungseinheit über einen bestimmten Zeitraum einer sehr hohen Keimdichte ausgesetzt ist, wie sie normalerweise nicht in der Umgebung von gelagerten Verpackungseinheiten vorkommt.

Der Erfindung liegt somit die Aufgabe zugrunde, eine gattungsgemäße Testeinheit bzw. ein gattungsgemäßes Prüfverfahren zur Verfügung zu stellen, mit deren Hilfe die Prüfung einer Endpackung nach einer praxisüblichen Lagerung, die auch Wochen oder Monate betragen, kann, auf einfache Weise erfolgen kann, ohne dass durch die Prüfung die Barrierewirksamkeit der Endpackung aufgehoben werden muss. Ferner liegt der Erfindung die Aufgabe zugrunde; eine Sterilisierverpackungseinheit zur Verfügung zu stellen, die, obwohl sie zumindest teilweise eine starre Außenhülle aufweist, zur Durchführung des erfindungsgemäßen Verfahrens geeignet ist.

Die Aufgabe bezüglich der Testeinheit wird durch die Merkmale des Anspruchs 1 gelöst. Die Testeinheit weist eine Matrixeinheit sowie ein hermetisch verschlossenen Behältnis mit einer Flüssigkeit auf. Die Matrixeinheit weist zumindest eine trockene Matrix zur Aufnahme eines Nährmediums auf oder besteht ausschließlich aus dieser trockenen Matrix. Die Matrix besitzt die Eigenschaft, Flüssigkeit binden, d.h. diese entgegen der Schwerkraft halten, zu können. Ohne eine entsprechende "Aktivierung" der Testeinheit enthält die Matrixeinheit aber noch kein vollständiges Nährmedium, d.h. die Matrixeinheit ist noch nicht für ein Wachstum von möglichen Keimen vollständig vorbereitet. Vielmehr geschieht dies erst dadurch, dass die Flüssigkeit auf die Matrix gegeben wird. Für diesen, in der Regel manuell vorzunehmenden Vorgang, sind das Behältnis und die Matrixeinheit derartig miteinander verbunden, dass die Flüssigkeit bei einem Öffnen des Behältnisses in Kontakt mit der Matrix gebracht werden kann. In der Regel wird die Flüssigkeit nach dem Öffnen des Behältnisses in Kontakt mit der Matrix gelangen, ohne dass dazu eine weitere diesbezügliche Handhabung der Testeinheit erforderlich ist. Das Öffnen des Behältnisses findet durch eine mechanische Einwirkung auf das Behältnis statt.

Indem die Flüssigkeit auf die Matrix gelangt, wird die Testeinheit aktiviert. Von diesem Zeitpunkt an, den der Anwender selbst bestimmen kann, bietet die Matrixeinheit eine Wachstumsgrundlage für Keime. Erfindungsgemäß ist vorgesehen, dass die zu prüfende Sterilisierverpackungseinheit nicht geöffnet wird, um die Testeinheit zu aktivieren. So kann z.B. dann, wenn es sich bei der Sterilisierverpackungseinheit um eine Papierverpackung handelt, die mechanische Einwirkung auf das Behältnis durch die Papierverpackung hindurch ausgeübt werden. Entsprechendes ist bei einer anderen flexiblen Verpackung möglich. Wenn sich nach einer entsprechenden Auswertung der Matrixeinheit zeigt, dass ein Keimwachstum, z.B. durch Koloniebildung, stattgefunden hat, so kann daraus geschlossen werden, dass diese Keime zum Zeitpunkt der Aktivierung der Testeinheit sich bereits in der Sterilisierverpackungseinheit befunden haben.

Mit der erfindungsgemäßen Testeinheit kann somit eine Endpackung nach einer Lagerungsdauer von z.B. zwei Monaten auf eine mögliche Rekontamination ihres Inhalts in ihrem praxisüblichen Zustand getestet werden, wobei die Barrierewirksamkeit der Endpackung nicht aufgehoben werden muss, d.h. durch den Prüfvorgang selbst keine Rekontaminationsgefahr gegeben ist.

Bei dem Behältnis handelt es sich vorzugsweise um eine Ampulle. Diese kann durch ein4 einfaches Zerdrücken zerstörbar sein. Dazu ist ferner vorzugsweise vorgesehen, dass das Behältnis bzw. die Ampulle mit einer Plastikhülle umgeben ist. Eine von einer Plastikhülle umgebene Ampulle ist an sich bekannt. Die Plastikhülle dient dazu, dass bei einem manuellen Zerdrücken einer Glasampulle keine Verletzungsgefahr besteht und Glassplitter aufgefangen werden. Ferner kann erfindungsgemäß die Plastikhülle auch dazu dienen, das Behältnis mit der Matrixeinheit zu verbinden. Vorzugsweise sind zwischen dem Behältnis und der Matrixeinheit Mittel angeordnet, die dazu dienen, die Flüssigkeit zur Matrix zu leiten. Auch diese Mittel können durch eine entsprechende schlauchartige Ausbildung eines Abschnitts der Plastikhülle auf einfache Weise gegeben sein. Es können auch eine Rinne oder ein Schlauch vorgesehen sein, um die Flüssigkeit zur Matrix zu leiten.

Insbesondere kann die Matrix ein so genannter Nährkarton sein. Bei einem solchen, an sich bekannten Nährkarton handelt es sich um Karton, der zwar in trockener Form einen Teil oder alle für Keimwachstum erforderlichen Nährstoffe enthält; solange der Nährkarton jedoch nicht mit einer entsprechenden Flüssigkeit, beispielsweise einfachem Wasser oder einer physiologischen Kochsalzlösung, benetzt wird, ist der Nährkarton noch nicht gebrauchsfertig. Der trockene Nährkarton, der insbesondere scheibenförmig sein kann, ist über lange Zeit, beispielsweise ein Jahr lang, haltbar.

Alternativ kann in dem Behältnis auch ein flüssiges, vollständiges Nährmedium enthalten sein.

Auf der Matrix kann als weiterer Bestandteil der Matrixeinheit ein Membranfilter angeordnet sein. Ein solcher keimdichter Membranfilter, auf dem die Keime statt auf der Matrix wachsen, ist denkbar, um gegenüber dem Wachstum von Keimen auf dem Nährkarton verbesserte Nachweisbedingungen für Keimkolonien zu erreichen. Der Membranfilter kann beispielsweise durch ein Haftmittel oder auch durch einen Wulst am Rand des Nährkartons an diesem befestigt sein.

Vorzugsweise befindet sich die Matrixeinheit in einer thermostabilen Nährbodenschale.

Die Matrixeinheit und das mit ihr verbundene Flüssigkeitsbehältnis können zur Stabilisierung auf einem gemeinsamen Träger aus einem geeigneten thermostabilen Material angeordnet sein.

Die Aufgabe bezüglich der Sterilisierverpackungseinheit, die zumindest teilweise eine starre Außenhülle aufweist, wird durch die Merkmale des Anspruchs 9 gelöst. Bei einer Sterilisierverpackungseinheit, die insgesamt oder teilweise eine starre Außenhülle bzw. Wandung aufweist und insbesondere eine Containerverpackung sein kann, kann das Behältnis einer in der Sterilisierverpackungseinheit positionierten Testeinheit in der Regel nicht durch Druck auf die Außenhülle der Sterilisierverpackungseinheit zerstört werden. Daher ist erfindungsgemäß vorgesehen, dass die Sterilisierverpackungseinheit an einer Innenseite der starren Außenhülle Halterungsmittel zur Befestigung einer Testeinheit aufweist. Die Testeinheit besitzt eine Matrixeinheit mit einer trockenen Matrix, die Flüssigkeit binden kann und zur Aufnahme eines Nährmediums vorgesehen ist. Ferner weist die Testeinheit ein hermetisch verschlossenes Behältnis auf, welches so beschaffen und mit der Matrixeinheit verbunden ist, dass durch mechanische Einwirkung auf das Behältnis dieses geöffnet und eine darin befindliche Flüssigkeit in Kontakt mit der Matrix gebracht werden kann. Eine solche Testeinheit ist oben beschrieben worden. Ferner weist die Sterilisierverpackungseinheit mindestens ein Betätigungsmittel auf, das so an der starren Außenhülle befestigt ist, dass es sich durch die Außenhülle hindurch erstreckt, wobei die Art der Befestigung keimdicht ist. Das Betätigungsmittel besitzt einen Abschnitt, der dazu dient, wenn eine Testeinheit von den Halterungsmitteln gehalten wird, die zur Öffnung des Behältnisses der Testeinheit vorgesehene mechanische Einwirkung auf das Behältnis auszuüben. Dazu kann der Anschlagabschnitt innerhalb der Sterilisierverpackungseinheit durch Betätigung des Betätigungmittels von außen bei ungeöffneter Sterilisierverpackungseinheit verschoben werden.

Das Betätigungs- bzw. die Betätigungsmittel können insbesondere einen Gewindebolzen aufweisen. Dabei ist der Anschlagabschnitt ein Teil des Gewindebolzens oder mit diesem verbunden, so dass durch ein Drehen des Gewindebolzens der Anschlagabschnitt innerhalb der Sterilisierverpackungseinheit verschoben werden kann.

Der Gewindebolzen kann insbesondere Teil einer Schraube sein. Grundsätzlich kann vorgesehen sein, solche Verpackungseinheiten und insbesondere Containerverpackungen so, wie es oben beschrieben ist, auszurüsten, um sie je nach Bedarf mit einer entsprechenden Testeinheit gemäß Anspruch 11 bestücken und dadurch hinsichtlich einer Wirksamkeit der Sterilisierverpackungseinheit gegenüber einer Rekontamination von enthaltenem Sterilisiergut überprüfen zu können, ohne dass dazu die Barrierewirksamkeit der Sterilisierverpackungseinheit aufgehoben werden muss. Denn nach dem Öffnen des Behältnisses und Aktivierung der Testeinheit erfolgt die Kultivierung, ohne dass die Containerverpackung zuvor geöffnet wird. Die Testeinheit kann nach ihrer Verwendung verworfen und auf einfache Weise durch eine neue Testeinheit ersetzt werden. Vorzugsweise ist die aktive Fläche der Matrixeinheit der Testeinheit etwa so groß wie die Grundfläche der Sterilisierverpackungseinheit. Natürlich sind auch andere Mechanismen zur Zerstörung des Behältnisses einer Testeinheit möglich, die in einer zumindest teilweise starren Sterilisierverpackungseinheit befestigt ist. Die Schraube kann gleichzeitig durch ihre Stellposition einen Indikator dafür liefern, ob die Sterilisierverpackungseinheit mit einer Testeinheit bestückt ist.

Die Aufgabe bezüglich des Verfahrens wird durch die Merkmale des Anspruchs 12 gelöst. Eine Testeinheit gemäß Anspruch 1 wird in die Sterilisierverpackungseinheit gegeben bzw., wenn es sich um ein starre Steritisierverpackungseinheit handelt, in dieser befestigt. Anschließend wird die Sterilisierverpackungseinheit einem Sterilisationsvorgang unterzogen. Danach wird die Sterilisierverpackungseinheit über einen Zeitraum unter Umgebungsbedingungen gelagert. Insbesondere können die Umgebungsbedingungen und der Zeitraum solcher Art sein, wie sie in der Praxis vorkommen. So werden beispielsweise in Krankenhäusern Einwegmaterial, wie Tupfer und Kompressen, oder wiederverwendbare Güter über einen Zeitraum von Wochen bis hin zu Monaten gelagert. Es ist aber auch möglich, dass es sich bei den Umgebungsbedingungen um besondere, zur speziellen Prüfung der Sterilisierverpackungseinheit vorgesehene Umgebungsbedingungen handelt. Insbesondere können diese hinsichtlich einer Rekontamination ungünstigere Bedingungen sein, als sie üblicherweise in der Praxis gegeben sind. Nach einer erfolgten Lagerung, also zu dem Zeitpunkt, zu dem geprüft werden soll, ob eine Rekontamination stattgefunden hat, wird das Behältnis durch mechanische Einwirkung geöffnet und die Flüssigkeit in Kontakt mit der Matrix gebracht. Dies kann bei einer starren Sterilisierverpackungseinheit wie oben beschrieben mittels von außen zugänglicher Betätigungsmittel erfolgen. Ab diesem Zeitpunkt können Keime, die sich auf der Matrixeinheit befinden, wachsen. Die Sterilisierverpackungseinheit wird dazu Vermehrungsbedingungen von Keimen ausgesetzt, um die auf der Matrixeinheit vorhandenen Keime zu kultivieren. Zu den Vermehrungsbedingungen gehören z.B. eine für die Vermehrung zu erwartender Keime günstige Temperatur sowie eine günstige Gasatmosphäre. In der Regel wird eine Bebrütung in einem Inkubator stattfinden. Danach wird die Sterilisierverpackungseinheit geöffnet, und Keime, die sich in oder auf der Matrixeinheit vermehrt haben, werden ermittelt. Insbesondere kann dabei als Maß der Wirksamkeit der Sterilisierverpackungseinheit gegen Rekontamination die Anzahl von auf der Matrixeinheit gewachsenen Kolonien, von denen jede auf einen sie auslösenden Keim zurückgeführt wird, bestimmt werden.

Labormäßige Ausstattung ist zur Anwendung des Verfahrens bzw. der Testeinheit lediglich in einem geringen Umfang erforderlich. Denn die Testeinheit ist so beschaffen, dass man bei ihrer Verwendung ohne labormäßige Ausstattung zur Nährmediumherstellung auskommt.

Sofern nach dem Sterilisationsvorgang, von dessen ordnungsgemäßem Ablauf ausgegangen wird bzw. dessen ordnungsgemäßer Ablauf mittels der oben beschriebenen Testkeimindikatoren geprüft worden ist, eine Kontamination der Matrixeinheit durch Eintritt von Keimen in die Sterilisierverpackungseinheit und Ansiedlung auf der Matrixeinheit stattgefunden hat, sind die Keime wie oben beschrieben kultiviert worden. Wenn auf der Matrixeinheit sich vermehrende Keime gefunden werden, ist dies ein klarer Beleg dafür, dass in die Sterilisierverpackungseinheit nach dem Sterilisationsvorgang Keime eingedrungen sind. Dies kann darauf zurückzuführen sein, dass die Sterilisierverpackungseinheit grundsätzliche Mängel aufweist, oder es kann auch darauf zurückzuführen sein, dass die Umgebungsbedingungen, wie z.B. Luftkeimgehalt und Nässe, in Bezug auf eine Vermeidung von Rekontamination sehr ungünstig waren.

Die Testeinheit kann insbesondere anstelle von Sterilisiergut in die Sterilisierverpackungseinheit gegeben werden. Eine solche Sterilisierverpackungseinheit könnte Teil einer zu sterilisierenden Charge von Sterilisiergut sein. Nach praxisüblicher Lagerung der Charge des Sterilisiergutes könnte dann die oben beschriebene Prüfung vorgenommen werden, wobei das Ergebnis der Prüfung auf die gesamte Charge des Sterilisiergutes übertragen werden kann. Damit ist eine besondere Qualitätssicherung ermöglicht. Solch eine Überprüfung könnte insbesondere dann sinnvoll sein, wenn das für eine Sterilitätsgewährleistung vorgesehene Verfallsdatum abgelaufen ist, es sich jedoch um sehr wertvolles Sterilisiergut handelt und eine Resterilisation aufwändig bzw. schwierig ist. Auch nach einer in Bezug auf eine mögliche Rekontamination relativ risikoreichen Handhabung von verpacktem Sterilisiergut kann ein solcher Test durchgeführt werden.

Weiterhin eignet sich das erfindungsgemäße Verfahren vorzugsweise dazu, eine grundsätzliche Prüfung einer Sterilisierverpackungseinheit vorzunehmen. Insbesondere bei dieser Art der Anwendung des erfindungsgemäßen Verfahrens können die Umgebungsbedingungen und der Zeitraum der Lagerung der Sterilisierverpackungseinheit vorgegeben sein.

Bei der verwendeten Testeinheit kann es sich um eine der oben beschriebenen Ausführungsformen der Testeinheit handeln.

Gegebenenfalls kann es auch sinnvoll sein, eine zu prüfende Sterilisierverpackungseinheit vor dem Sterilisationsvorgang außer mit der Testeinheit auch mit Sterilisiergut zu beladen. Damit kann eine hohe Sicherheit erreicht werden, eine unerkannte Rekontamination des Sterilisiergutes zu vermeiden.

Im Folgenden wird die Erfindung anhand eines Ausführungsbeispiels näher erläutert, wobei auf die Figuren Bezug genommen wird. Es zeigen:
Fig. 1 schematisch in perspektivischer Ansicht eine Testeinheit zur Prüfung einer Sterilisierverpackungseinheit hinsichtlich der Barrierewirksamkeit der Sterilisierverpackungseinheit bezüglich einer Rekontimination und
Fig. 2 schematisch eine Containerverpackung mit einer Testeinheit.

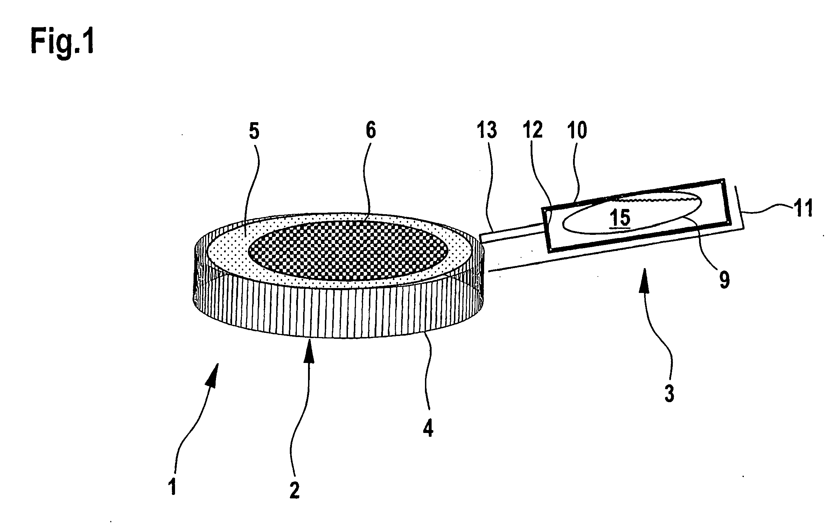

Die Testeinheit gemäß Fig. 1 ist mit dem Bezugszeichen 1 bezeichnet. Die Testeinheit 1 besteht aus zwei Teilelementen 2 und 3. Das Teilelement 2 weist eine thermostabile Nährbodenschale 4 auf. In der Nährbodenschale 4 befindet sich eine Nährkartonscheibe 5. Auf der Nährkartonscheibe 5 ist ein Membranfilter 6 befestigt. Die Befestigung wird mittels eines im Randbereich des Membranfilters vorgesehenen, nicht gezeigten Haftmittels bewirkt.

Das Teilelement 3 weist eine Glasampulle 9 auf. Die Ampulle 9 befindet sich in einer Plastikhülle 10. Die Ampulle 9 und die Plastikhülle 10 sind auf einem Träger 11 aus einem geeigneten Material angeordnet, der mit einer Neigung zu dem Teilelement 2 seitlich an diesem befestigt ist.

Die Plastikhülle 10 weist einen Auslass 12 auf. Der Auslass 12 mündet in eine Rinne 13, die sich zwischen der Plastikhülle 10 und der Nährbodenschale 4 erstreckt. In der Ampulle 9 befindet sich Wasser 15.

Durch Druck auf die Plastikhülle 10 und damit auf die Ampulle 9 kann diese zerstört werden, wodurch das Wasser 15 durch den Auslass 12 der Plastikhülle 10 in die Rinne 13 gelangt. Aufgrund der Neigung des Teilelements 3 fließt das Wasser 15 auf die Nährkartonscheibe 5 in der Nährbodenschale 4. Indem die Nährkartonscheibe 5 mit dem Wasser 15 benetzt wird, wird sie zu einem gebrauchsfertigen Nährboden, auf dem Keime grundsätzlich wachsen können. Die Nährkartonscheibe 5 und der Membranfilter 6, auf dem sich mögliche Keime ansiedeln und vermehren, können entsprechend gewünschter Anforderungen ausgewählt werden.

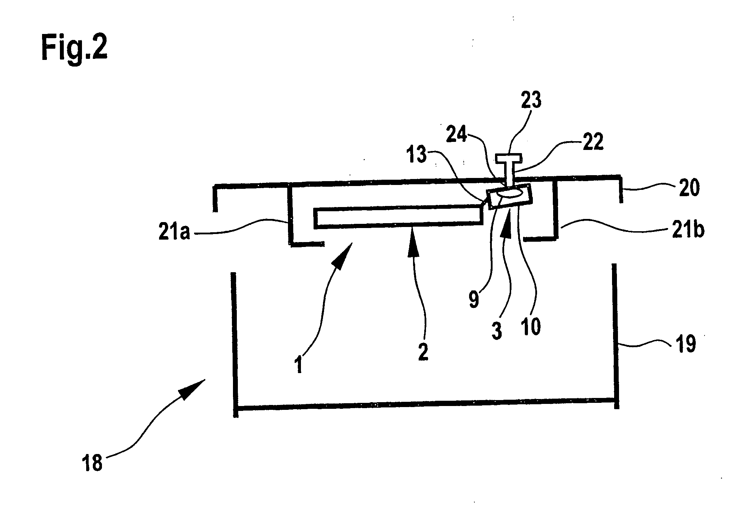

Die Containerverpackung gemäß Fig. 2 ist mit dem Bezugszeichen 18 bezeichnet und weist eine Wanne 19 und einen Deckel 20 auf. Der Deckel 20 ist in einem von der Wanne 19 abgehobenen Zustand dargestellt und kann keimdicht auf die Wanne 19 zur Verschließung der Containerverpackung 18 aufgesetzt werden. An der Innenseite des Deckels 20 ist eine Testeinheit 1 mittels einer Halterung mit zwei schematisch dargestellten Halterungselementen 21 a und 21 b befestigt. Die Testeinheit 1 entspricht im Wesentlichen der Testeinheit gemäß Fig. 1. Gleiche Teile sind in den Figuren mit gleichen Bezugszeichen bezeichnet.

Durch den Deckel 20 hindurch ist ein Gewindebolzen 22 einer Schraube 23 so mittels eines nicht gezeigten Gewindes in dem Deckel 20 befestigt, dass bei einem entsprechend weiten Eindrehen der Schraube 23 der Bolzen 22 mit seinem als Anschlagabschnitt 24 bezeichneten freien Ende auf die Plastikhülle 10 der Testeinheit 1 und auf die Glasampulle 9 drückt. Mittels der Schraube 23 kann die Glasampulle 9 zerstört und damit bewirkt werden, dass in der Glasampulle 9 enthaltenes Wasser 15 durch die Rinne 13 in das Teilelement 2 und somit auf eine (in Fig. 2 nicht gezeigte) Nährkartonscheibe 5 läuft.

Mittels der Schraube 23, die auch durch ein entsprechendes ähnliches Betätigungsmittel ersetzt werden könnte, kann somit die Ampulle 9 zerstört werden, wenn die Containerverpackung 18 geschlossen ist.

## Patentansprüche

1. Keimdurchlässige Testeinheit (1) zur Prüfung einer zur Sterilisation von Sterilisiergut, insbesondere medizinischem Sterilisiergut, vorgesehenen Sterilisierverpackungseinheit (18) hinsichtlich der Wirksamkeit der Sterilisierverpackungseinheit (18), eine mikrobielle Rekontamination des Sterilisiergutes nach dessen erfolgter Sterilisation zu verhindern,
**dadurch gekennzeichnet, dass** die Testeinheit (1) eine Matrixeinheit (5,6) mit einer trockenen Matrix (5), die Flüssigkeit binden kann und zur Aufnahme eines Nährmediums dient, und ein hermetisch verschlossenes Behältnis (9) mit einer Flüssigkeit (15) aufweist,
wobei die Matrix (5) und die Flüssigkeit (15) so beschaffen sind, dass durch Aufgabe der Flüssigkeit (15) auf die Matrix (5) diese in den Zustand versetzt wird, dass sie ein vollständiges Nährmedium enthält, und
wobei das Behältnis (9) so beschaffen und mit der Matrixeinheit (5,6) verbunden ist, dass durch mechanische Einwirkung auf das Behältnis (9) dieses geöffnet und die Flüssigkeit (15) in Kontakt mit der Matrix (5) gebracht werden kann.

2. Testeinheit nach Anspruch 1,
**dadurch gekennzeichnet, dass** das Behältnis eine Ampulle (9) ist.

3. Testeinheit nach Anspruch 1 oder 2,
**dadurch gekennzeichnet, dass** das Behältnis (9) mit einer Plastikhülle (10) versehen ist.

4. Testeinheit nach einem der vorhergehenden Ansprüche,
**dadurch gekennzeichnet, dass** zwischen dem Behältnis (9) und der Matrixeinheit (5,6) Mittel (13) vorgesehen sind, um die Flüssigkeit (15) zur Matrix (5) zu leiten.

5. Testeinheit nach einem der vorhergehenden Ansprüche,
**dadurch gekennzeichnet, dass** die Matrix ein Nährkarton (5) ist.

6. Testeinheit nach einem der Ansprüche 1 bis 4,
**dadurch gekennzeichnet, dass** die Flüssigkeit (15) das vollständige Nährmedium ist.

7. Testeinheit nach einem der vorhergehenden Ansprüche,
**dadurch gekennzeichnet, dass** die Matrixeinheit (5,6) einen Membranfilter (6) aufweist, der auf der Matrix (5) angeordnet ist.

8. Testeinheit nach einem der vorhergehenden Ansprüche,
**dadurch gekennzeichnet, dass** sich die Matrixeinheit (5,6) in einer thermostabilen Nährbodenschale (4) befindet.

9. Sterilisierverpackungseinheit (18), die zumindest teilweise eine starre Außenhülle (19,20) und ferner an der starren Außenhülle (19,20) angeordnete Halterungsmittel (21 a,21 b) zur Befestigung einer Testeinheit (1) aufweist, insbesondere eine Containerverpackung,
**dadurch gekennzeichnet, dass** die Halterungsmittel (21a,21b) an einer Innenseite der starren Außenhülle (19,20) angeordnet sind,
es sich bei der Testeinheit um eine Testeinheit (1) nach Anspruch 1 handelt,
und die Sterilisierverpackungseinheit (18) ferner mindestens ein an der starren Außenhülle (20) befestigtes und sich keimdicht durch die Außenhülle (20) hindurch erstreckendes, von außen betätigbares Betätigungsmittel (23) mit einem zum Anschlagen vorgesehenen Abschnitt (24) aufweist, welches derartig ausgebildet ist, dass, wenn von den Halterungsmitteln (21a,21b) eine besagte Testeinheit (1) gehalten wird, bei Betätigung des Betätigungsmittels (23) durch eine Verschiebung des Anschlagabschnitts (24) innerhalb der Sterilisierverpackungseinheit- (18) die zur Öffnung des, Behältnisses (9) vorgesehene mechanische Einwirkung auf das Behältnis (9) ausgeübt werden kann.

10. Sterilisierverpackungseinheit nach Anspruch 9,
**dadurch gekennzeichnet, dass** das Betätigungsmittel (23) einen Gewindebolzen (22) aufweist, der so, vorzugsweise in einem Deckel (20) der Sterilisierverpackungseinheit (18), angeordnet ist, dass durch ein Eindrehen des Gewindebolzens (22) der Anschlagabschnitt gegen das Behältnis (9) drückt.

11. Sterilisierverpackungseinheit nach Anspruch 9 oder 10,
**dadurch gekennzeichnet, dass** in ihr eine Testeinheit (1) nach einem der Ansprüche 1 bis 8 befestigt ist

12. Verfahren zur Prüfung einer zur Sterilisation von Sterilisiergut, insbesondere medizinischem Sterilisiergut, vorgesehenen Sterilisierverpackungseinheit (18) hinsichtlich der Wirksamkeit der Sterilisierverpackungseinheit (18), eine mikrobielle Rekontamination des Sterilisiergutes nach dessen erfolgter Sterilisation zu verhindern, **dadurch gekennzeichnet, dass**
- in die Sterilisierverpackungseinheit (18) eine Testeinheit (1) nach Anspruch 1 gegeben wird, welche eine Matrixeinheit (5,6) mit einer trockenen Matrix (5) zur Aufnahme eines Nährmediums und ein hermetisch verschlossenes Behältnis (9) mit einer Flüssigkeit (15) aufweist, wobei die Matrix (5) und die Flüssigkeit (15) so beschaffen sind, dass durch Aufgabe der Flüssigkeit (15) auf die Matrix (5) diese in den Zustand versetzt wird, dass sie ein Nährmedium enthält, und wobei das Behältnis (9) so beschaffen und mit der Matrixeinheit (5,6) verbunden ist, dass durch mechanische Einwirkung auf das Behältnis (9) dieses geöffnet und die Flüssigkeit (15) in Kontakt mit der Matrix (5) gebracht werden kann,
- die Sterilisierverpackungseinheit (18) einem Sterilisationsvorgang unterzogen wird,
- die Sterilisierverpackungseinheit (18) unter Umgebungsbedingungen über einen Zeitraum gelagert wird,
- das Behältnis (9) durch mechanische Einwirkung geöffnet und die Flüssigkeit (15) in Kontakt mit der Matrix (5) gebracht wird,
- die Sterilisierverpackungseinheit (18) Vermehrungsbedingungen von Keimen zu einer Kultivierung von auf der Matrixeinheit (5,6) vorhandenen Keimen ausgesetzt wird,
- die Keime, die sich auf der Matrixeinheit (5,6) vermehrt haben, ermittelt werden.

13. Verfahren nach Anspruch 12,
**dadurch gekennzeichnet, dass** es sich bei der Testeinheit (1) um eine Testeinheit nach einem der Ansprüche 2 bis 8 handelt.

14. Verfahren nach Anspruch 12 oder 13,
**dadurch gekennzeichnet, dass** bei der Lagerung der Sterilisierverpackungseinheit (18) die Umgebungsbedingungen und der Zeitraum vorgegeben werden.

15. Verfahren nach einem der Ansprüche 12 bis 14,
**dadurch gekennzeichnet, dass** die Sterilisierverpackungseinheit (18) vor dem Sterilisationsvorgang außer mit der Testeinheit (1) auch mit Sterilisiergut beladen wird.

## Claims

1. Testing unit (1) permeable to microbes for testing a sterilization packaging unit (18) which is provided for the sterilization of objects to be sterilized, in particular medical objects to be sterilized, with regard to the efficacy of the sterilization packaging unit (18) in preventing microbial recontamination of the sterilized objects after they have been sterilized,
**characterized in that** the testing unit (1) comprises a matrix unit (5,6) having a dry matrix (5), which can bind liquid and serves to accommodate a nutrient medium, and comprises a hermetically sealed receptacle (9) containing a liquid (15),
wherein the matrix (5) and the liquid (15) are configured in such a manner that by applying the liquid (15) on to the matrix (5) the matrix is placed in the condition of containing a complete nutrient medium, and
wherein the receptacle (9) is configured and connected to the matrix unit (5,6) such that by virtue of mechanical effect upon the receptacle (9), the receptacle can be opened and the liquid (15) can be brought into contact with the matrix (5).

2. Testing unit as claimed in claim 1,
**characterized in that** the receptacle is an ampoule (9).

3. Testing unit as claimed in claim 1 or 2,
**characterized in that** the receptacle (9) is provided with a plastics casing (10).

4. Testing unit as claimed in any one of the preceding claims,
**characterized in that** means (13) for guiding the liquid (15) to the matrix (5) are provided between the receptacle (9) and the matrix unit (5,6).

5. Testing unit as claimed in any one of the preceding claims,
**characterized in that** the matrix is a nutrient board (5).

6. Testing unit as claimed in any one of claims 1 to 4,
**characterized in that** the liquid (15) is the complete nutrient medium.

7. Testing unit as claimed in any one of the preceding claims,
**characterized in that** the matrix unit (5,6) comprises a membrane filter (6) which is disposed on the matrix (5).

8. Testing unit as claimed in any one of the preceding claims,
**characterized in that** the matrix unit (5,6) is located in a thermostable solid nutrient medium dish (4).

9. Sterilization packaging unit (18) which comprises at least partially a rigid outer casing (19, 20) and further comprises holding means (21a,21b) which are disposed on the rigid outer casing (19, 20) in order to secure a testing unit (1), in particular a container package,
**characterized in that** the holding means (21a,21b) are disposed on an inner side of the rigid outer casing (19,20),
the testing unit is a testing unit (1) as claimed in claim 1,
and the sterilization packaging unit (18) further comprises at least one actuating means (23) which is attached to the rigid outer casing (20), extends in a microbe-tight manner through the outer casing (20), can be actuated from the outside, has a portion (24) which is provided as a stop, and is formed in such a manner that, when a said testing unit (1) is held by the holding means (21a,21b), the mechanical effect provided for opening the receptacle (9) can be exerted upon the receptacle (9) during actuation of the actuating means (23) by virtue of a displacement of the stop portion (24) within the sterilization packaging unit (18).

10. Sterilization packaging unit as claimed in claim 9,
**characterized in that** the actuating means (23) comprises a threaded bolt (22) which is disposed, preferably in a cover (20) of the sterilization packaging unit (18), such that by screwing in the threaded bolt (22) the stop portion presses against the receptacle (9).

11. Sterilization packaging unit as claimed in claim 9 or 10,
**characterized in that** a testing unit (1) as claimed in any one of claims 1 to 8 is secured in said sterilization packaging unit.

12. Method of testing a sterilization packaging unit (18) which is provided for the sterilization of objects to be sterilized, in particular medical objects to be sterilized, with regard to the efficacy of the sterilization packaging unit (18) in preventing microbial recontamination of the sterilized objects after they have been sterilized, **characterized in that**
- a testing unit (1) as claimed in claim 1 is inserted into the sterilization packaging unit (18) and comprises a matrix unit (5,6) having a dry matrix (5) for accommodating a nutrient medium, and a hermetically sealed receptacle (9) containing a liquid (15), wherein the matrix (5) and the liquid (15) are configured in such a manner that by applying the liquid (15) on to the matrix (5) the matrix is placed in the condition of containing a nutrient medium, and wherein the receptacle (9) is configured and connected to the matrix unit (5,6) such that by virtue of mechanical effect exerted upon the receptacle (9), the receptacle can be opened and the liquid (15) can be brought into contact with the matrix (5),
- the sterilization packaging unit (18) is subjected to a sterilization process,
- the sterilization packaging unit (18) is stored for a period of time under ambient conditions,
- the receptacle (9) is opened by means of mechanical effect and the liquid (15) is brought into contact with the matrix (5),
- the sterilization packaging unit (18) is subjected to conditions in which microbes multiply for cultivation of microbes which are present on the matrix unit (5,6),
- the microbes which have multiplied on the matrix unit (5,6) are observed.

13. Method as claimed in claim 12,
**characterized in that** the testing unit (1) is a testing unit as claimed in any one of claims 2 to 8.

14. Method as claimed in claim 12 or 13,
**characterized in that** the ambient conditions and the period of time are predetermined for the storage of the sterilization packaging unit (18).

15. Method as claimed in any one of claims 12 to 14,
**characterized in that** prior to the sterilization process the sterilization packaging unit (18) is loaded with objects to be sterilized in addition to the testing unit (1).

## Revendications

1. Unité de vérification (1) perméable aux micro-organismes pour le contrôle d'une unité d'emballage et de stérilisation (18) prévue pour la stérilisation de produits à stériliser, en particulier de produits médicaux à stériliser, afin de vérifier l'efficacité de l'unité d'emballage et de stérilisation (18) et d'empêcher une recontamination microbienne des produits à stériliser après la stérilisation de ceux-ci,
**caractérisée en ce que** l'unité de vérification (1) est une unité de matrice (5, 6) avec une matrice sèche (5) qui peut retenir un liquide et qui sert à recevoir un milieu nutritif, et présente un récipient (9) fermé hermétiquement avec un liquide (15),
dans laquelle la matrice (5) et le liquide (15) sont tels que le dépôt du liquide (15) sur la matrice (5) amène celle-ci à un état dans lequel elle contient un milieu nutritif complet, et
dans laquelle le récipient (9) est construit et relié à l'unité de matrice (5, 6) de telle manière que le récipient (9) soit ouvert par une action mécanique et le liquide (15) amené en contact avec la matrice (5).

2. Unité de vérification selon la revendication 1, **caractérisée en ce que** le récipient est une ampoule (9).

3. Unité de vérification selon la revendication 1 ou 2, **caractérisée en ce que** le récipient (9) est doté d'une enveloppe en plastique (10).

4. Unité de vérification selon l'une des revendications précédentes, **caractérisée en ce qu'**il est prévu entre le récipient (9) et l'unité de matrice (5, 6) des moyens (13) pour amener le liquide (15) jusqu'à la matrice (5).

5. Unité de vérification selon l'une des revendications précédentes, **caractérisée en ce que** la matrice est un carton pour milieu nutritif (5).

6. Unité de vérification selon l'une des revendications 1 à 4, **caractérisée en ce** le liquide (15) est le milieu nutritif complet.

7. Unité de vérification selon l'une des revendications précédentes, **caractérisée en ce** l'unité de matrice (5, 6) présente un filtre à membrane (6) disposé sur la matrice (5).

8. Unité de vérification selon l'une des revendications précédentes, **caractérisée en ce** l'unité de matrice (5, 6) se trouve dans une boîte pour milieu nutritif (5) thermostable.

9. Unité d'emballage et de stérilisation (18) comprenant au moins une enveloppe extérieure rigide (19, 20) et des moyens de fixation (21a, 21b) disposés sur l'enveloppe extérieure rigide (19, 20) pour la fixation d'une unité de vérification (1), en particulier un emballage de conteneur,
**caractérisée en ce que** les moyens de fixation (21a, 21b) sont disposés sur une face intérieure de l'enveloppe extérieure rigide (19, 20),
l'unité de vérification est une unité de vérification (1) selon la revendication 1,
et l'unité d'emballage et de stérilisation (18) possède en outre au moins un moyen d'actionnement (23) fixé à l'enveloppe extérieure rigide (20) et s'étendant de façon étanche aux micro-organismes à travers l'enveloppe extérieure (20), pouvant être actionné de l'extérieur, avec une partie servant de butée (24), qui est conformé de telle sorte que lorsqu'une unité de vérification (1) décrite est retenue par les moyens de fixation (21a, 21b), lors de l'actionnement du moyen d'actionnement (23), la translation de la partie servant de butée (24) à l'intérieur de l'unité d'emballage et de stérilisation (18) puisse exercer sur le récipient (9) l'action mécanique prévue pour ouvrir le récipient (9).

10. Unité d'emballage et de stérilisation selon la revendication 9, **caractérisée en ce que** le moyen d'actionnement (23) possède un goujon fileté (22), de préférence disposé dans un couvercle (20) de l'unité d'emballage et de stérilisation (18), de telle sorte que la rotation du goujon fileté (22) appuie la partie servant de butée contre le récipient (9).

11. Unité d'emballage et de stérilisation selon la revendication 9 ou 10, **caractérisée en ce qu'**une unité de vérification (1) selon l'une des revendications 1 à 8 est fixée à l'intérieur

12. Procédé pour le contrôle d'une unité d'emballage et de stérilisation (18) prévue pour la stérilisation de produits à stériliser, en particulier de produits médicaux à stériliser, afin de vérifier l'efficacité de l'unité d'emballage et de stérilisation (18) et d'empêcher une recontamination microbienne des produits à stériliser après la stérilisation de ceux-ci, **caractérisé en ce que** :
- il est prévu dans l'unité d'emballage et de stérilisation (18) une unité de vérification (1) selon la revendication 1, qui contient une unité de matrice (5, 6) avec une matrice sèche (5) destinée à recevoir un milieu nutritif et un récipient (9) fermé hermétiquement et contenant un liquide (15), la matrice (5) et le liquide (15) étant conçus de telle sorte que lorsque le liquide (15) est déposé sur la matrice (5), celle-ci est amenée dans un état dans lequel elle contient un milieu nutritif, le récipient (9) étant conformé et relié à l'unité de matrice (5, 6) de telle sorte qu'une action mécanique sur le récipient (9) ouvre celui-ci et amène le liquide (15) en contact avec la matrice (5),
- l'unité d'emballage et de stérilisation (18) est soumise à une opération de stérilisation,
- l'unité d'emballage et de stérilisation (18) est stockée dans les conditions ambiantes pendant un certain temps,
- le récipient (9) est ouvert par une action mécanique et le liquide (15) est mis en contact avec la matrice (5),
- l'unité d'emballage et de stérilisation (18) est exposée à des conditions de multiplication des micro-organismes afin de cultiver des micro-organismes présents sur l'unité de matrice (5, 6),
- les micro-organismes qui se sont multipliés sur l'unité de matrice (5, 6) sont déterminés.

13. Procédé selon la revendication 12, **caractérisé en ce que** l'unité de vérification (1) est une unité de vérification selon l'une des revendications 2 à 8.

14. Procédé selon la revendication 12 ou 13, **caractérisé en ce que** les conditions d'environnement et la durée du stockage de l'unité d'emballage et de stérilisation (18) sont prédéterminées.

15. Procédé selon l'une des revendications 12 à 14, **caractérisé en ce que** l'unité d'emballage et de stérilisation (18) est remplie avant l'opération de stérilisation non seulement avec l'unité de vérification (1) mais aussi avec des produits à stériliser.
